(19) Europäisches Patentamt
European Patent Office
Office européen des brevets

(11) **EP 1 643 126 B1**

(12) **EUROPEAN PATENT SPECIFICATION**

(45) Date of publication and mention
of the grant of the patent:
**21.05.2008 Bulletin 2008/21**

(51) Int Cl.:
*F04B 43/04* (2006.01)     *F04B 19/00* (2006.01)
*F04B 35/00* (2006.01)     *F04B 35/04* (2006.01)
*H01L 41/09* (2006.01)     *H01L 41/193* (2006.01)
*A61B 5/021* (2006.01)     *A61H 23/04* (2006.01)

(21) Application number: **05020604.4**

(22) Date of filing: **21.09.2005**

(54) **Air pump, pump system, and electronic blood pressure monitor using the same**

Luftpumpe, Pumpsystem, und deren Verwendung in einem elektronischen Blutdruckmessgerät

Pompe à air, système de pompage, et leur utilisation dans un appareil électronique de mesure de pression sanguine

(84) Designated Contracting States:
**DE ES FR GB IT NL**

(30) Priority: **22.09.2004 JP 2004275578**

(43) Date of publication of application:
**05.04.2006 Bulletin 2006/14**

(73) Proprietor: **Omron Healthcare Co., Ltd.**
**Kyoto 615-0084 (JP)**

(72) Inventors:
• **Karo, Hiromichi**
**c/o Omron Healthcare Co., Ltd.**
**Ukyo-ku**
**Kyoto-shi**
**Kyoto 615-0084 (JP)**
• **Sano, Yoshihiko**
**c/o Omron Healthcare Co., Ltd.**
**Ukyo-ku**
**Kyoto-shi**
**Kyoto 615-0084 (JP)**
• **Kishimoto, Hiroshi**
**c/o Omron Healthcare Co., Ltd.**
**Ukyo-ku**
**Kyoto-shi**
**Kyoto 615-0084 (JP)**
• **Miyata, Kiichiro**
**c/o Omron Healthcare Co., Ltd.**
**Ukyo-ku**
**Kyoto-shi**
**Kyoto 615-0084 (JP)**
• **Eda, Kenji**
**c/o Omron Healthcare Co., Ltd.**
**Ukyo-ku**
**Kyoto-shi**
**Kyoto 615-0084 (JP)**
• **Tanaka, Takahide**
**c/o Omron Healthcare Co., Ltd.**
**Ukyo-ku**
**Kyoto-shi**
**Kyoto 615-0084 (JP)**

(74) Representative: **Kilian, Helmut**
**Wilhelms, Kilian & Partner**
**Patentanwälte**
**Eduard-Schmid-Strasse 2**
**81541 München (DE)**

(56) References cited:
**EP-A- 0 363 672**     **EP-A- 0 760 233**
**EP-A- 1 285 626**     **WO-A-03/081762**
**WO-A-20/04076859**     **US-A1- 2004 008 853**
**US-A1- 2004 097 854**

**Description**

Background of the Invention

Field of the Invention

The present invention relates to an air pump and a pump system, and more particularly, to an electronic blood pressure monitor and a massager using the air pump or the pump system.

Description of the Related Art

When a blood pressure monitor for household use is used to measure blood pressure, an air bag (a cuff) is attached to a part of a human body such as an upper arm. The cuff is expanded by pressure from an air pump, so as to pressurize the human arteries and to obtain an arterial wave. Abloodpressure value is then determined from the arterial wave.

There is an increasing demand for small-sized blood pressure monitors, in favor of high portability and storability. In this trend, air pumps that are contained in blood pressure monitors are also expected to be smaller.

Examples of air pumps that have been employed for conventional blood pressure monitors are as follows.

Japanese Patent No. 2551757 discloses a small-sized pump that feeds and discharges air by periodically changing the volume of a diaphragm in synchronization with the rotation of the driving axis. The diaphragm is linked to a driving body that is decentered by the rotation of the output axis of a motor.

Japanese Patent No. 3373558 discloses a small-sized pump system that comprises an actuator rod that is decentered in the radial direction of a motor by the rotation of the output axis of the motor, and a diaphragm unit that is compressed and expanded by the reciprocating movement of the actuator rod, thereby functioning as a pump.

Japanese Patent Application Laid-Open No. 2003-193979 discloses a diaphragm pump that employs an electrostriction polymer actuator for driving the diaphragm.

In each of the pumps disclosed in Japanese Patent Nos. 2551757 and 3373558, and Japanese Patent Application Laid-Open No. 2003-193979, however, a motor or an actuator that is provided outside the diaphragm is used to change the volume of the diaphragm that generates pressure from a volume change of the air chamber. There are the following problems with such a structure:

> 1) The rotational movement of the motor needs to be converted to vertical or horizontal reciprocating movement, and energy loss is caused during the operation of converting the rotational movement to reciprocating movement.
> 2) A component for converting the rotational movement to reciprocating movement and a component for deforming the diaphragm are required, and extra room for placing those components outside the diaphragm is also required.
> 3) Noise is generated by a rotating motor and the component that is driving for converting the direction of movement.
> 4) The current consumption of the motor is high.

Also, Japanese Patent Application Laid-Open Nos. 2001-269375 and 2003-250842 disclose a compressing device that compresses an object with an actuator having conductive elastic electrodes formed on both sides of an elastic sheet made of an insulating elastic material, and a human body assisting device that provides assisting force in the same direction as the movement of a joint of a human body.

An air pump according to the preamble of claim 1 is known from US 2004/0008853 A1.

Summary of the Invention

In view of the conventional techniques described above, an object of the present invention is to provide an air pump with a simple structure. Another object of the present invention is to provide a compact electronic blood pressure monitor that is equipped with air pumps. Yet another obj ect of the present invention is to provide a compact massager that is equipped with air pumps.

In order to achieve the above-describedobject, an air pump according to the present invention is as defined in claim 1 and has:

> a first hollow actuator that has elastomer or polymer members that can expand and contract in response to voltage application, and electrodes for applying voltage to the elastomer or polymer members;
> a suction unit that sucks fluid into the hollow actuator and a discharge unit that discharges fluid to the outside of the hollow actuator upon expansion or contraction of said first actuator, characterized by
> a second hollow actuator that has elastomer or polymer members that can expand and contract in response to voltage application, and electrodes for applying voltage to the elastomer or polymer members, said first and second actuators being arranged such that a contraction of said first actuator causes an expansion of said second actuator and a contraction of said second actuator causes an expansion of said first actuator.

Here, materials such as dielectric elastomer and electrostriction polymer (such as silicone resin, acrylic resin, and polyurethane, which are electroactive plastics with high striction) can be employed as the elastomer or polymer members that can expand and contract when a voltage is applied. Particularly, materials with electroactive polymer artificial muscles (EPAM) are preferable.

With this structure, a small-sized air pump with a simple structure can be provided without a motor.

Further, the air pump preferably comprises a pump chamber that is surrounded by the actuator, a first housing member to which the suction unit is attached, and a second housing member to which the discharge unit is attached, wherein the volume of the pump chamber is varied by expansion and contraction of the actuator, and the pressurized fluid is discharged to the outside.

Using this structure, the actuator also serves as a pump wall, and expands and contracts to change the volume of the pump chamber. Thus, a pumping function can be provided with a simple structure that does not require a motor.

Further, preferably the first housing member has a first check valve that allows fluid to flow from the outside of the actuator only into the pump chamber; and the second housing member has a second check valve that allows fluid to flow from the pump chamber only to the outside of the actuator.

Using the two check valves in this structure, an air pump with high pumping efficiency can be provided.

Further, the actuator preferably has a varied extent of expansion and contraction depending on the magnitude of an applied voltage.

Further, voltage or frequency to be applied to the actuator is preferably controlled to control the discharge flow rate and discharge pressure of fluid to be discharged from the pump chamber.

In this structure, the voltage to be applied to the actuator or the frequency of applied voltage are controlled so as to change the stroke and the frequency of the actuator for deforming the diaphragm. Thus, the discharge flow rate and discharge pressure can be controlled without a change in the structure of the air pump.

Further, the air pump comprises a restoration mechanism that generates restoring force against expansion and contraction of the actuator.

Further, the restoration mechanism is preferably an elastic member that is supported by the first housing member and the second housing member in such a manner that the elastic member penetrates the pump chamber of the actuator.

Further, the restoration mechanism is preferably an elastic member that is supported by the first housing member and the second housing member in such a manner that the elastic member covers the outer surface of the actuator.

Further, the restoration mechanism is preferably a U-shaped flexible member that is provided outside the actuator, and is linked to both ends of the actuator.

In this structure, the restoration mechanism generates restoring force against the actuator that is compressed or expanded. Thus, a highly responsive pumping operation can be performed.

Further, preferably the actuator has first elastomer or polymer members that reduce the volume of the pump chamber by voltage application, and second elastomer or polymer members that increase the volume of the pump chamber by voltage application; and the volume of the pump chamber is varied by alternately applying voltage to the first elastomer or polymer members and the second elastomer or polymer members, thereby discharging pressurized fluid to the outside.

In this structure, the actuator can increase and reduce the volume of the pump chamber by voltage application. Accordingly, the number of components can be reduced, and higher performance in assembling can be achieved, without a spring or a flexible member for generating restoring force.

Further, the first housing member or the second housing member preferably covers the outer surface of the actuator so as to protect the elastomer or polymer members and guide the actuator at the time of expansion and contraction.

In this structure, the housing member protects the actuator and guides the actuator when the actuator expands and contracts. Thus, an air pump that performs stable pumping operations can be provided.

Further, in the air pump, preferably a plurality of the actuators are connected in series, and suction and discharge are conducted through the connecting parts; the total length of the plurality of connected actuators in the direction of expansion and contraction is constant; and in response to contraction or expansion of one of the actuators, another one of the actuators expands or contracts.

In this structure, a plurality of actuators is connected in series, and the total length of the actuators is made constant. In this manner, the force generated by one of the actuators at the time of contraction can be used as the restoring force for another actuator that is compressed. Accordingly, there is no need to employ a restoration mechanism such as a spring or a flexible member, and the number of components can be reduced.

Further, the actuator preferably has a check valve at an end to which the suction unit or the discharge unit is attached.

In this structure, the actuator itself also functions as a check valve. Accordingly, the number of components can be reduced.

A pump system according to the present invention comprises a plurality of any of the above-described air pumps, with discharge units of the air pumps being connected to one another, the phases of voltages to be applied to actuators of the air pumps being shifted from one another.

In this structure, the discharge units of the plurality of actuators are connected in parallel, and the same flow rate of fluid as that to be discharged by a single pump is discharged by the pumps. Also, the operating timings of the actuators are shifted from one another, so as to reduce the ripple (the pressure change) caused by the discharged fluid. Accordingly, when the pump system is employed for a blood pressure monitor, highly accurate measurement can be performed.

Further, the phases are preferably shifted $2\pi/n$ from one another, with n being the number of air pumps.

With this structure, the ripple can be made even smaller.

Further, an electronic blood pressure monitor according to the present invention comprises: a fluid bag that is filled with fluid such as air and is wound around a living body; a cuff that externally fixes the fluid bag; any of the above-described air pumps or any of the above-described pump systems that introduces fluid into the fluid bag and pressurizes the fluid bag; a pressure sensor that detects the inner pressure of the fluid bag; and operating means that performs an operation for blood pressure measurement based on the detected inner pressure.

In this structure, a complicated mechanism such as a motor or a clutch is not necessary, and a component for converting the direction of movement from rotational movement to reciprocating movement is not required either. Accordingly, an electronic blood pressure monitor with a simple structure can be provided. Also, there is no driving noise of a motor. Accordingly, the driving noise of the air pump can be reduced, as there are no components for converting the direction of movement. Furthermore, operation can be performed with a lower current than a current required for driving a motor.

In accordance with the present invention, an air pump is provided with a simple structure. Also, a small-sized, lightweight electronic blood pressure monitor is provided. Furthermore, a small-sized, lightweight massager is provided.

BRIEF DESCRIPTION OF THE DRAWINGS

FIGS. 1A and 1B are cross-sectional views illustrating a suction state and a discharge state of an air pump in accordance with a first embodiment;

FIGS. 2A and 2B are schematic views illustrating deformations when a voltage is applied to the actuator;

FIGS. 3A and 3B are schematic views illustrating the structure of EPAM;

FIGS. 4A and 4B are schematic cross-sectional views of an air pump in accordance with a second embodiment;

FIGS. 5A and 5B are schematic cross-sectional views of an air pump in accordance with a third embodiment;

FIGS. 6A and 6B are schematic cross-sectional views of an air pump in accordance with a fourth embodiment which is an embodiment of the invention;

FIGS. 7A through 7C are schematic cross-sectional views of air pumps in accordance with fifth and sixth embodiments;

FIGS. 8A and 8B are schematic cross-sectional views of an air pump in accordance with a seventh embodiment;

FIGS. 9A and 9B are schematic cross-sectional views of an air pump in accordance with an eighth embodiment which is an embodiment of the invention;

FIG. 10A is a schematic cross-sectional view of a pump system in accordance with a ninth embodiment;

FIG. 10B illustrates ripples due to pressure variations;

FIG. 11 is a block diagram illustrating the hardware structure of an electronic blood pressure monitor in accordance with a tenth embodiment;

FIG. 12 is a flowchart of the basic operation of the electronic blood pressure monitor in accordance with the tenth embodiment;

FIG. 13 is an external perspective view illustrating the fundamental structure of a massager;

FIG. 14 is a block diagram illustrating the structure of the massager of Fig. 13;

FIG. 15 is a cross-sectional view of the main components of the massager;

FIG. 16A is a rear view of the back of the massager, illustrating an example of the arrangement of air pumps and exhaust valves; and

FIG. 16B is a vertical cross-sectional view of the back of the massager, taken along the center line and seen from the right side.

Detailed Description of the Preferred Embodiments

Hereinafter, preferred embodiments of the present invention will be described in detail with reference to the drawings and examples. It should be noted that the sizes, the materials, the shapes, the functions, and the arrangement of the components of the embodiments do not limit the scope of the invention, unless otherwise specifically mentioned. In the following description, the material, the shape, and the functions of each component are the same as those first described unless otherwise specified.

[First Embodiment]

(Summary of Air Pump)

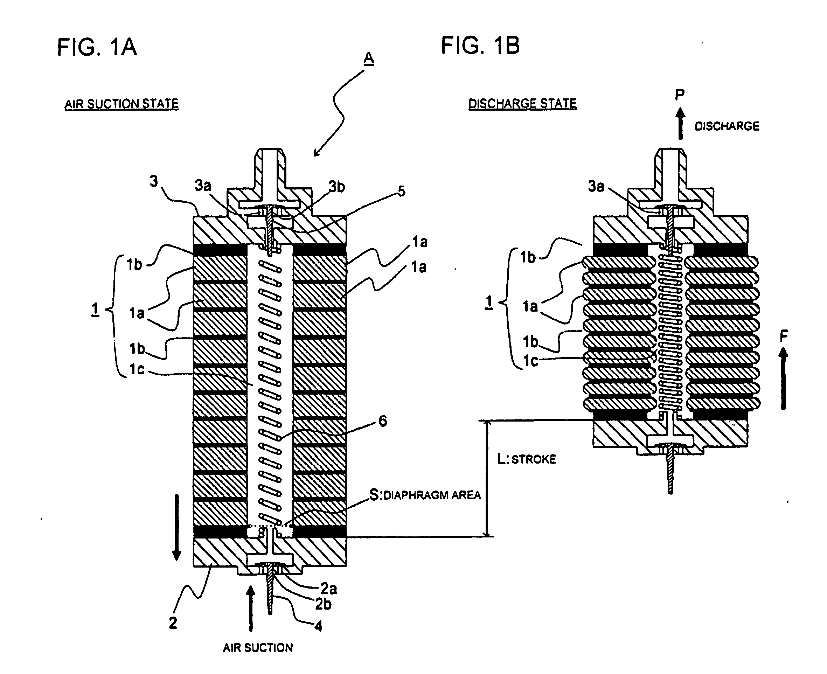

The structure and principles of an air pump in accordance with a first embodiment are described referring to FIGS. 1A and 1B. FIGS. 1A and 1B are cross-sectional views illustrating an air inlet state and an air outlet state of the air pump in accordance with the first embodiment.
An air pump A includes an actuator 1 that functions as a part of a diaphragm or the entire diaphragm, housing members 2 and 3 provided on the suction (air inlet) side and the discharging (air outlet) side of the actuator 1, a first check valve 4 provided on the housing member 2 on the suction side, a second check valve 5 provided on the housing member 3 on the discharging side, and a spring 6 provided inside the actuator 1.
The actuator 1 has elastomer or polymer members 1a that can expand and contract with a voltage, and electrodes 1b that are provided for applying a voltage to the elastomer or polymer members 1a. The elastomer or polymer members 1a and the electrodes 1b each have a cylindrical shape (a ring-like shape), and are alternately stacked, thereby forming the hollow actuator 1 that has a pump chamber 1c. Here, the pump chamber 1c is surrounded by the actuator 1, the housing member 2 on the suction side, and the housing member 3 on the discharging side.
The elastomer or polymer members 1a are preferably formed with electroactive polymer artificial muscles (EPAM) that will be described later. In each of the following embodiments, EPAM is used for the actuator 1.
The housing members 2 and 3 are provided at two openings of the cylindrical actuator 1. The housing member 2 includes a suction unit 2a that sucks fluid from the outside of the air pump A into the pump chamber 1c of the hollow actuator 1, and a support hole 2b that is formed to support the later-mentioned check valve 4 by virtue of the pressure difference between the outside and the pump chamber 1c. The housing member 3 includes a discharge unit 3a that discharges fluid from the pump 1c to the outside of the air pump A, and a support hole 3b that is formed to support the later-mentioned check valve 5 by virtue of the pressure difference between the pump chamber 1c and the outside.
The first check valve 4 is slidably supported by the support hole 2b, and allows fluid to pass from the outside of the actuator 1 only toward the pump chamber 1c. The second check valve 5 is slidably supported by the support hole 3b, and allows fluid to pass from the pump chamber 1c only to the outside of the actuator 1. Here, a "check valve" is a valve that opens when the pressure in one of the two regions sandwiching the valve is higher than the pressure in the other one of the two regions, but remains closed when the pressure in the other one of the two regions is higher than the pressure in the one of the two regions.
The spring 6 that is made of an elastic material and is an example of a restoration mechanism for generating restoring force against expansion and contraction is attached to the centers of the surfaces of the housing members 2 and 3 to which the actuator 1 is also attached, in such a manner that the spring 6 is supported by the housing members 2 and 3. More specifically, the spring 6 is provided inside the cylindrical actuator 1 in such a manner that the spring 6 penetrates the pump chamber 1c and is interposed between the first housing member and the second housing member. The spring 6 generates restoring force for returning from the situation in which the diaphragm contracts due to an applied voltage to the original state.
As shown in FIG. 1A, in the air pump A, a gap is formed between the first check valve 4 and the housing member 2 so as to fill the pump chamber 1c with fluid sucked through the suction unit 2a when the fluid is sucked from the outside into the pump chamber 1c by driving the actuator 1.
As shown in FIG. 1B, when a compression load F is applied to the actuator 1 by virtue of the reciprocating motion (expansion and contraction) of the actuator 1 itself, the volume of the pump chamber 1c varies, and the check valve 4 blocks the suction unit 2a. At the same time, the fluid sucked into the pump chamber 1c is pressurized, and is discharged from the discharge unit 3a to the outside.
The pressure flow rate (the discharge flow rate) Q (ml/min) of the air pump A, and the load F (N/cm$^2$) and the discharge pressure P (mmHg = $1.332 \times 10^{-2}$N/cm$^2$) required at the time of compression are determined by the following equations:
$$Q = \eta_P \times 760/(760+P) \times V \times f \times 60 \quad \dots \quad (1)$$

$$F = F_0 + (S \times P \times 1.332 \times 10^{-2}) \quad \dots \quad (2)$$

$$P = 760 \times (V_0/V_1)^{\kappa} - 760 \quad \dots \quad (3)$$

where:

$\eta_p$ is the pump efficiency;
$V(ml) = V_0$ (the volume of the pump chamber at the time of expansion) - $V_1$ (the volume of the pump chamber at the time of compression) is the volume change;
$f(Hz)$ is the frequency;
$S(cm^2)$ is the diaphragm area;
$F_0(N/cm^2)$ is the diaphragm deformation load (at the atmospheric pressure); and
$\kappa$ is the coefficient of adiabatic change.

According to the equations (1) through (3), the discharge pressure P is determined by the compression ratio of $V_0$ to $V_1$, and can be controlled by changing the stroke L between the top dead center and the bottom dead center of the diaphragm shown in FIGS. 1A and 1B. The pressure flow rate Q can be controlled by adjusting the volume change V and the frequency f, as well as the discharge pressure P.

(Summary of Actuator Used in Air Pump)

A small-sized air pump with a simple structure that does not require a motor can be provided by employing, as diaphragms, an actuator having elastomer or polymer members that can expand and contract due to voltage application, and electrodes that are provided for applying a voltage to the elastomer or polymer members, instead of elastic bodies made of elastic materials such as TPE, NBR, CR, EPDM, or fluorocarbon rubber.

The actuator 1 using EPAM has a variable length (the stroke L) that expands and contracts according to the magnitude of an applied voltage. Accordingly, the voltage and the frequency of a signal inputted to drive the actuator are controlled so as to readily change the pressure flow rate and the discharge pressure of the fluid to be discharged from the pump chamber.

Since the design of the actuator can be set according to the required pressure and the required flow rate, a high discharge pressure with a low driving force can be realized by reducing the inner diameter area of the actuator or by extending the stroke of the actuator (or increasing the voltage to be applied) to increase the compression ratio. In a case where a high pressure flow rate is required, the volume of the pump chamber of the actuator should be increased, or the driving frequency of the actuator or the voltage to be applied should be increased.

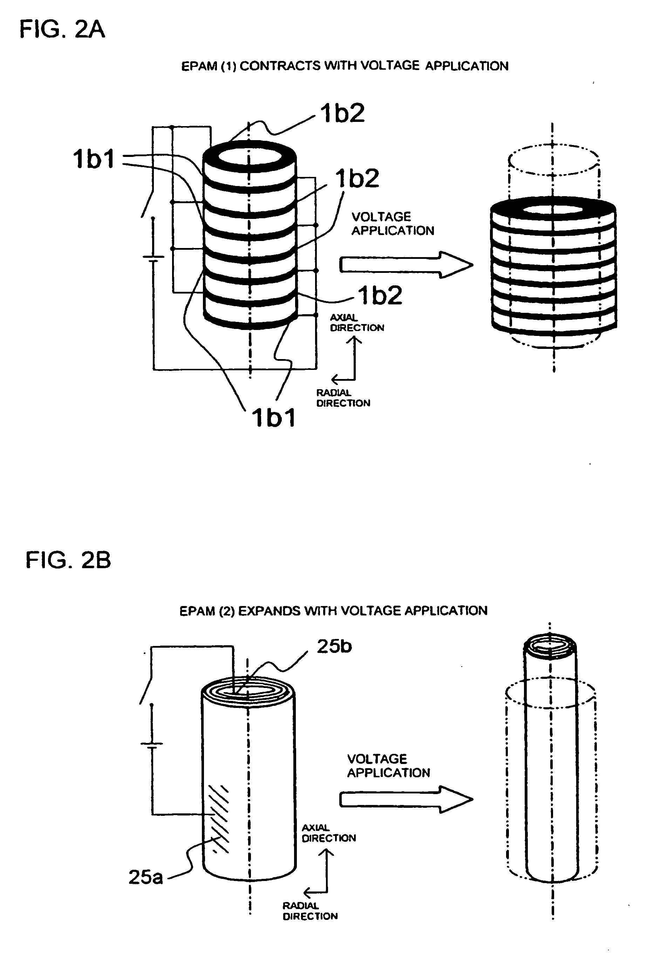

FIGS. 2A and 2B are schematic views illustrating deformations when a voltage is applied to the actuator used in a preferred embodiment of the present invention.

As shown in FIG. 2A, the actuator 1 in accordance with this embodiment has electrodes 1b1 and 1b2 at both ends of the EPAM (1) in the axial direction. Additionally, in this embodiment, electrodes 1b1 and 1b2 are alternately provided between the EPAMs (1). When a voltage V is applied between the electrodes 1b1 and 1b2, the actuator 1 contracts in the axial direction and expands in the radial direction. Accordingly, the actuator 1 tries to contract in its longitudinal direction. As a result, the distance between the housing members 2 and 3 becomes shorter, and the fluid inside the pump chamber 1c is discharged.

With the above described actuator 1, the air pump A in accordance with this embodiment can deform the actuator 1 as a diaphragm from the suction state (FIG. 1A) to the discharge state (FIG. 1B), without a complicated mechanism such as a motor or a clutch.

When the voltage application is suspended, the deformed actuator 1 returns to the original shape by virtue of the restoring force of the spring 6 provided at the center of the actuator 1. Thus, the actuator 1 returns from the contraction state to the suction state.

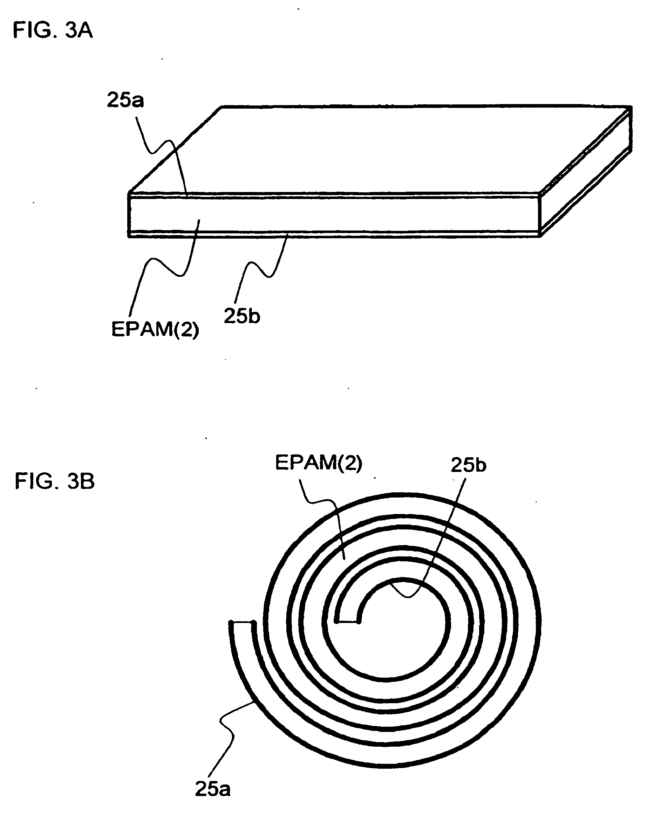

As shown in FIG. 3A, an actuator 11 in accordance with another embodiment of the present invention has film-like EPAM (2) having stretchable electrodes 25a and 25b attached to both surfaces. The EPAM (2) is rolled once or several times, as shown in FIG. 3B. When the EPAM (2) is rolled, an insulating material (not shown) is rolled together is the EPAM (2), so as to insulate each two neighboring electrodes. Accordingly, as the voltage V is applied between the electrodes 25a and 25b as shown in FIG. 2B, the actuator 11 contracts in the radial direction and expands in the axial direction. As a result, the actuator 11 tries to expand in its longitudinal direction. The distance between the housing members 2 and 3 then becomes longer, and the fluid is sucked into the pump chamber 1c.

By using the above-described actuator 11, an air pump A' in accordance with another embodiment of the present embodiment can deform the actuator 11 as a diaphragm from the suction state (FIG. 1A) to the contraction state (FIG. 1B), without a complicated mechanism such as a motor or a clutch.

When the voltage application is suspended, the deformed actuator 11 returns to the original shape by virtue of the restoring force of the spring provided at the center of the actuator 11. Thus, the actuator 11 returns from the contraction state to the suction state.

Referring back to FIGS. 1A and 1B, the operation of the air pump in accordance with the first embodiment using the actuator 1 is described.

As shown in FIG. 1A, in a case where the voltage to be applied to the actuator 1 is OFF, the actuator 1 stands still at such a location that the volume of the pump chamber 1c is the largest. In this situation, the pressure is almost the same between the inside and the outside of the pump chamber 1c. Accordingly, the fluid introduced through the suction unit 2a flows into the pump chamber 1c via the gap between the check valve 4 and the housing member 2.

After that, when the voltage to be applied to the actuator 1 is turned ON, as shown in FIG. 1B, the actuator 1 contracts in its longitudinal direction, and the volume of the pump chamber 1c becomes smaller. At this point, the first check valve 4 closes, as the pressure inside the pump chamber 1c increases. Accordingly, the fluid flowing through the suction unit 2a is shut off. As the pump chamber 1c is compressed, the pressure inside the pump chamber 1c further increases and the second check valve 5 opens. As a result, the fluid is discharged to the outside through the discharge unit 3a.

When the voltage to be applied to the actuator 1 is again turned OFF, the actuator 1 expands by virtue of the restoring force of the spring 6 provided in the actuator 1. As a result, the volume of the pump chamber 1c increases, and the pressure in the pump chamber 1c drops. Accordingly, the first check valve 4 opens when the pressure in the pump chamber 1c becomes lower than the outside pressure, and the fluid flows from the outside into the pump chamber 1c.

With its reciprocating movement (expansion and contraction), the actuator 1 repeatedly switches between the states shown in FIGS. 1A and 1B, thereby changing the volume of the pump chamber 1c and the pressure in the pump chamber 1c. Thus, an air pump with a simple structure can be provided, without a power transmission device such as a motor or a gear.

With the above described air pump A in accordance with the first embodiment, a complicated mechanism such as a conventional motor or clutch is not necessary, and a component for converting the direction of movement from rotational movement to reciprocating movement is not required either. Accordingly, an air pump with a simple structure can be provided. Also, such an air pump is smaller and lighter than a conventional air pump with a motor.

With this air pump A, there is no driving noise of a motor. Accordingly, the driving noise of the air pump can be reduced, since there are no components for converting the direction of movement. Furthermore, the air pump A can operate with a lower current than a current required for driving a motor.

Compared with the case where the disk-like electrostriction polymer actuator of the prior art is employed as a diaphragm, the volume change of the pump chamber can be easily made greater.

[Second Embodiment]

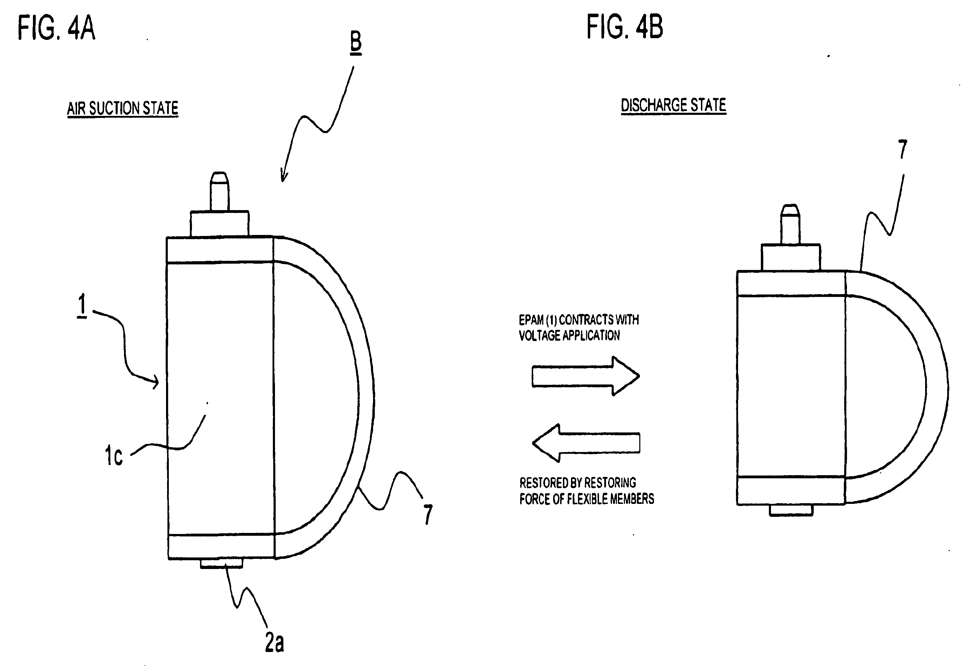

Referring now to FIGS. 4A and 4B, the structure of an air pump in accordance with a second embodiment is described.

An air pump B in accordance with the second embodiment differs from the air pump A of the first embodiment in that a U-shaped flexible member is externally joined to the ends of the actuator 1, instead of the spring 6, as a restoration mechanism for generating restoring force against contraction. In the following, explanation is focused on the differences between the first and second embodiments.

As shown in FIG. 4A, in a case where the voltage to be applied to the actuator 1 is OFF, the actuator 1 stands still at such a location that the volume of the pump chamber 1c becomes the largest. In this situation, the pressure is almost the same between the inside and the outside of the pump chamber 1c. Accordingly, the fluid introduced through the suction unit 2a flows into the pump chamber 1c through the gap between the check valve 4 and the housing member 2.

After that, when the voltage to be applied to the actuator 1 is turned ON, the actuator 1 contracts in its longitudinal direction, and the volume of the pump chamber 1c becomes smaller, as shown in FIG. 4B. At this point, the first check valve 4 closes, as the pressure inside the pump chamber 1c increases. Accordingly, the fluid flowing through the suction unit 2a is shut off. As the pump chamber 1c is compressed, the pressure inside the pump chamber 1c further increases and the second check valve 5 opens. As a result, the fluid is discharged to the outside through the discharge unit 3a.

When the voltage to be applied to the actuator 1 is again turned OFF, the actuator 1 expands in its longitudinal direction by virtue of the restoring force of the flexible member 7 externally attached to the actuator 1. As a result, the volume of the pump chamber 1c increases, and the pressure in the pump chamber 1c drops. Accordingly, the fluid flows from the outside into the pump chamber 1c.

With its reciprocating movement (expansion and contraction), the actuator 1 repeatedly switches between the states shown in FIGS. 4A and 4B, thereby changing the volume of the pump chamber 1c and the pressure in the pump chamber 1c. Thus, an air pump with a simple structure can be provided, without a power transmission device such as a motor or a gear.

With the above described air pump B in accordance with the second embodiment, a complicated mechanism such as a conventional motor or clutch is not necessary, and a component for converting the direction of movement from rotating movement to reciprocating movement is not required either. Accordingly, an air pump with a simple structure can be provided. Also, such an air pump is smaller and lighter than a conventional air pump with a motor.

With this air pump B, there is no driving noise of a motor. Accordingly, the driving noise of the air pump can be reduced, since there are no components for converting the direction of movement. Furthermore, the air pump B can operate with a lower current than a current required for driving a motor.

Compared with the case where the disk-like electrostriction polymer actuator of the prior art is employed as a diaphragm, the volume change of the pump chamber can be easily made greater.

Although the EPAM (1) shown in FIG. 2A is used for the actuator 1 in the second embodiment, the same effects as those of the second embodiment can be achieved by employing the EPAM (2) shown in FIG. 2B.

[Third Embodiment]

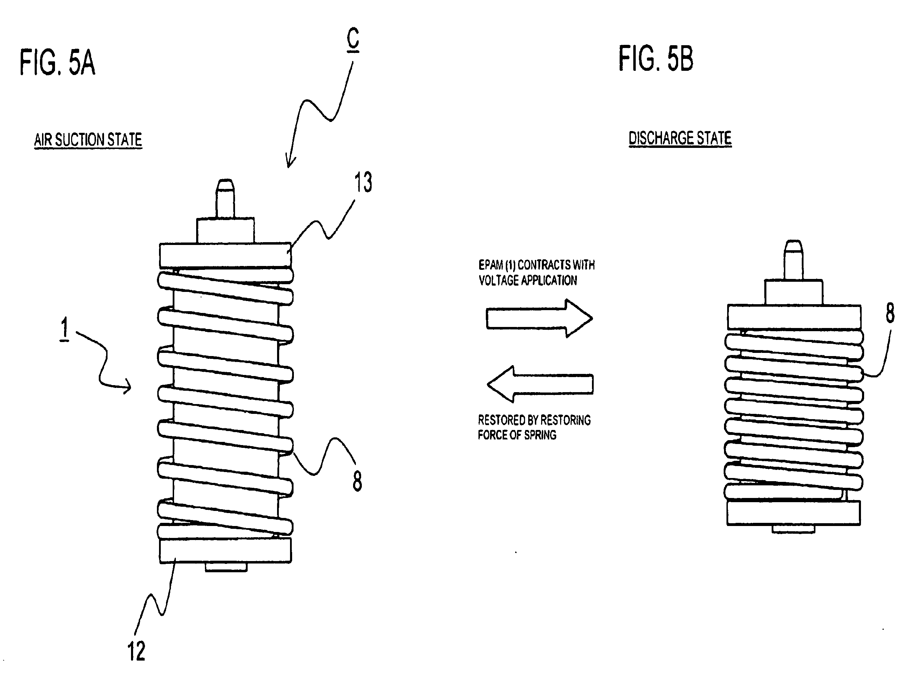

Referring now to FIGS. 5A and 5B, the structure of an air pump in accordance with a third embodiment is described.

An air pump C in accordance with the third embodiment differs from the air pump A of the first embodiment in that housing members 12 and 13 each having a greater diameter than the outer diameter of the actuator 1, and, as a restoration mechanism for generating the restoring force against expansion and contraction, a spring 8 that is an elastic member supported by the first housing member and the second housing member in such a manner as to cover the outer surface of the actuator 1 are provided. The main difference is that the spring 8 with a greater diameter than the outer diameter of the actuator 1 is provided outside the air pump C and between the housing members 12 and 13. In the following, explanation is focused on the differences between the first and third embodiments.

As shown in FIG. 5A, in a case where the voltage to be applied to the actuator 1 is OFF, the actuator 1 stands still at such a location that the volume of the pump chamber 1c becomes the largest.

After that, when the voltage to be applied to the actuator 1 is turned ON, the actuator 1 contracts in its longitudinal direction, and the volume of the pump chamber 1c becomes smaller, as shown in FIG. 5B.

When the voltage to be applied to the actuator 1 is again turned OFF, the actuator 1 expands in its longitudinal direction by virtue of the restoring force of the spring 8 externally attached to the actuator 1. As a result, the volume of the pump chamber 1c increases, and the pressure in the pump chamber 1c drops. Accordingly, the fluid flows from the outside into the pump chamber 1c.

With its reciprocating movement (expansion and contraction), the actuator 1 repeatedly switches between the states shown in FIGS. 5A and 5B, thereby changing the volume of the pump chamber 1c and the pressure in the pump chamber 1c. Thus, an air pump with a simple structure can be provided, without a power transmission device such as a motor or a gear.

With the above described air pump C in accordance with the third embodiment, a complicated mechanism such as a conventional motor or clutch is not necessary, and a component for converting the direction of movement from rotating movement to reciprocating movement is not required either. Accordingly, an air pump with a simple structure can be provided. Also, such an air pump is smaller and lighter than a conventional air pump with a motor.

With the spring 8 having a greater diameter than the diameter of the actuator 1, there is no need to provide a spring inside the pump chamber 1c, which facilitates the assembling of the device. Also, since a spring having a great restoring force can be used, the compressive force F can be increased.

With this air pump C, there is no driving noise of a motor. Accordingly, the driving noise of the air pump can be reduced, since there are no components for converting the direction of movement. Furthermore, the air pump C can operate with a lower current than a current required for driving a motor.

Compared with the case where the disk-like electrostriction polymer actuator of the prior art is employed as a diaphragm, the volume change of the pump chamber can be easily made greater.

Although the EPAM (1) shown in FIG. 2A is used for the actuator 1 in this embodiment, the same effects as those of this embodiment can be achieved by employing the EPAM (2) shown in FIG. 2B.

[Fourth Embodiment]

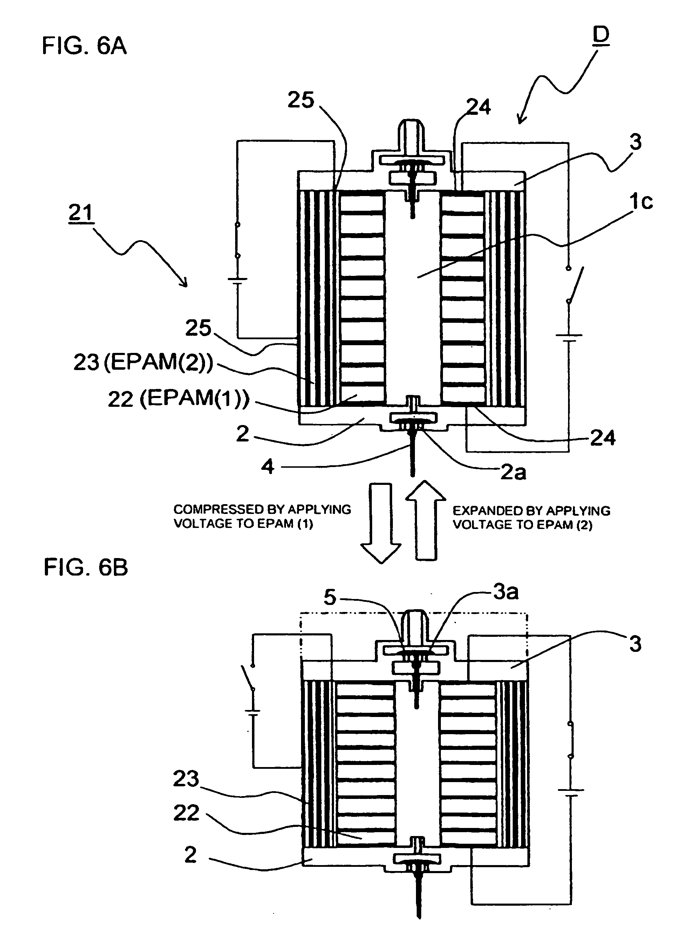

Referring now to FIGS. 6A and 6B, the structure of an air pump in accordance with a fourth embodiment is described.

An air pump D in accordance with the fourth embodiment differs from the air pump A of the first embodiment in that a spring is eliminated by employing an actuator 21 combining the EPAM (1) and the EPAM (2) shown in FIGS. 2A and 2B. In the following, explanation is focused on the differences in the structure, functions, and effects between the first and fourth embodiments, and explanation of the same components as those of the first embodiment is omitted.

FIGS. 6A and 6B are cross-sectional views of the actuator 21 in accordance with the fourth embodiment, which has EPAM 22 (EPAM (1)) and EPAM 23 (EPAM (2)), and pairs of electrodes 24 and 25 for applying a voltage to the EPAM 22 and the EPAM 23.

In the actuator 21 in accordance with the fourth embodiment, the EPAM (1) and the EPAM (2) used in the first embodiment shown in FIGS. 2A and 2B can be employed. In the fourth embodiment, the actuator 21 has a cylindrical shape (a columnar shape) . The EPAM 23 that is rolled up as shown in FIG. 2B is provided outside the actuator 21, and the cylindrical EPAM 22 as shown in FIG. 2A is provided inside the actuator 21.

In a case where the voltage to be applied to the EPAM 22 and the EPAM 23 of the actuator 21 is OFF, the actuator 21 stands still at such a location that the volume of the pump chamber 1c is constant. In this state, the pressure is almost the same between the outside and the inside of the pump chamber 1c. Accordingly, the fluid introduced through the suction unit 2a flows into the pump chamber 1c through the gap between the first check valve 4 and the housing member 2.

After that, a voltage is not applied between the electrodes 25, but is applied between the electrodes 24 (see FIGS. 6A and 6B) . The cylindrical EPAM 22 is then compressed in its axial direction and expands in its radial direction, as shown in FIG. 6B. Accordingly, the entire actuator 21 contracts vertically. Since the distance between the housing members 2 and 3 becomes shorter, the volume of the pump chamber 1c becomes smaller. At this point, the pressure in the pump chamber 1c increases, and the first check valve 4 closes. As a result, the fluid flowing through the suction unit 2a is shut off. Further, the pump chamber 1c is compressed, and the pressure in the pump chamber 1c increases accordingly. The second check valve 5 then opens, and the fluid is discharged to the outside through the discharge unit 3a.

After that, a voltage is not applied between the electrodes 24, but is applied between the electrodes 25 (see FIGS. 6A and 6B). The rolled EPAM 23 is then compressed in its radial direction and expands in its axial direction, as shown in FIG. 6A. Accordingly, the entire actuator 21 expands vertically. Since the distance between the housing members 2 and 3 becomes longer, the volume of the pump chamber 1c becomes larger. When the pressure in the pump chamber 1c becomes lower than the outside pressure, the first check valve 4 opens. As a result, the fluid flows from the outside into the pump chamber 1c.

As described above, the actuator 21 has the EPAM 22 that reduces the volume of the pump chamber 1c when a voltage is applied, and the EPAM 23 that increases the volume of the pump chamber 1c when a voltage is applied. A voltage is alternately applied to the EPAM 22 and the EPAM 23, so that the volume of the pump chamber 1c is varied. Thus, the pressurized fluid is discharged to the outside.

With its reciprocating movement (expansion and contraction), the actuator 21 repeatedly switches between the states shown in FIGS. 6A and 6B, thereby changing the volume and the pressure of the pump chamber 1c of the actuator 21. Thus, an air pump with a simple structure can be provided, without a power transmission device such as a motor or a gear.

With the above described air pump D in accordance with the fourth embodiment, a complicated mechanism such as a conventional motor or clutch is not necessary, and a component for converting the direction of movement from rotating movement to reciprocating movement is not required either. Accordingly, an air pump with a simple structure can be provided. Also, such an air pump is smaller and lighter than a conventional air pump with a motor.

As the EPAMs of two different types with different voltage application directions are employed, each voltage to be applied is suitably controlled. Accordingly, the actuator 21 can contract and expand without a spring. Thus, the number of components required can be reduced, and the device can be made smaller and lighter.

With this air pump D, there is no driving noise of a motor. Accordingly, the driving noise of the air pump can be reduced, since there are no components for converting the direction of movement. Furthermore, the air pump D can operate with a lower current than a current required for driving a motor.

Compared with the case where the disk-like electrostriction polymer actuator of the prior art is employed as a diaphragm, the volume change of the pump chamber can be easily made greater.

[Fifth Embodiment]

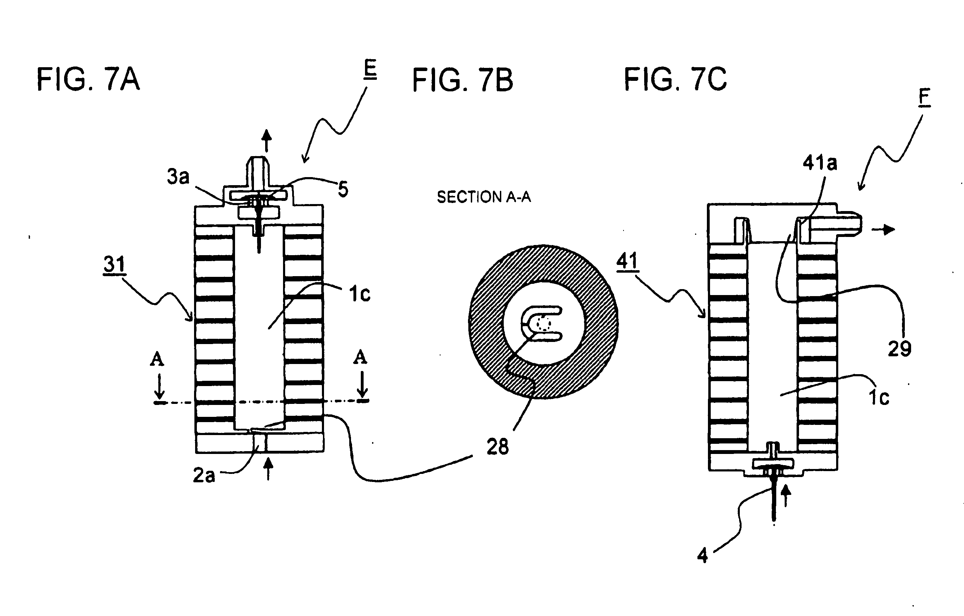

Referring now to FIGS. 7A and 7B, the structure of an air pump in accordance with a fifth embodiment is described.

An air pump E in accordance with the fifth embodiment differs from the air pump A of the first embodiment in that a leaf valve 28, instead of the first check valve 4, is integrally formed with the EPAM at the end of an actuator 31 to which the suction unit is attached. The valve material formed with the elastomer members constituting the EPAM is preferably a dielectric elastomer material such as silicon or polyurethane. In the following, explanation is focused on the differences in the structure, functions, and effects between the first and fifth embodiments, and explanation of the same components as those of the first embodiment is omitted.

As shown in FIG. 7A, in a case where the voltage to be applied to the actuator 31 is OFF, the actuator 31 stands still at such a location that the volume of the pump chamber 1c becomes the largest.

After that, when the voltage to be applied to the actuator 31 is turned ON, the actuator 31 contracts in its longitudinal direction, and the volume of the pump chamber 1c becomes smaller. At this point, the pressure in the pump chamber 1c increases, and the leaf valve 28 closes. As a result, the fluid flowing through the suction unit 2a is shut off. Further, the pump chamber 1c is compressed, and the pressure in the pump chamber 1c increases accordingly. The second check valve 5 then opens, and the fluid is discharged to the outside through the discharge unit 3a.

When the voltage to be applied to the actuator 31 is again turned OFF, the actuator 31 expands in its longitudinal direction by virtue of the restoring force of the spring (not shown) provided on the actuator 31. As a result, the volume of the pump chamber 1c increases, and the pressure in the pump chamber 1c drops. Accordingly, the leaf valve 28 opens, and the fluid flows from the outside into the pump chamber 1c.

Since one of the housing members is formed with the EPAM and the leaf valve is provided at the bottom portion as described above, the number of components can be reduced, and an air pump with a simpler structure can be provided.

Although the EPAM (1) shown in FIG. 2A is used for the actuator 31 in this embodiment, the same effects as the above can be achieved by employing the EPAM (2) shown in FIG. 2B. [Sixth Embodiment]

Referring now to FIG. 7C, the structure of an air pump in accordance with a sixth embodiment is described.

An air pump F in accordance with the sixth embodiment differs from the air pump A of the first embodiment in that a discharge valve 41a, instead of the second check valve 5, is integrally formed with the EPAM at an end of an actuator 41. The valve material formed with the elastomer members constituting the EPAM is preferably a dielectric elastomer material such as silicon or polyurethane. In the following, explanation is focused on the differences in the structure, functions, and effects between the first and sixth embodiments, and explanation of the same components as those of the first embodiment is omitted.

The discharge valve 41a having a smaller diameter than the outer diameter of the actuator 41 is provided at one end of the actuator 41. A cylindrical member 29 covers the discharge valve 41a to form a check valve.

In a case where the voltage to be applied to the actuator 41 in accordance with the sixth embodiment is OFF, the actuator 41 stands still at such a location that the volume of the pump chamber 1c becomes the largest.

After that, when the voltage to be applied to the actuator 41 is turned ON, the actuator 41 contracts in its longitudinal direction, and the volume of the pump chamber 1c becomes smaller accordingly. At this point, the pressure in the pump chamber 1c increases, and the first checkvalve 4 closes. As a result, the fluid flowing through the suction unit 2a is shut off. Further, the pump chamber 1c is compressed, and the pressure in the pump chamber 1c increases accordingly. The cylindrical member 29 is then pushed outward, and the fluid is discharged to the outside through the gap formed between the discharge valve 41a and the cylindrical member 29.

When the voltage to be applied to the actuator 41 is again turned OFF, the actuator 41 expands in its longitudinal direction by virtue of the restoring force of the spring (not shown) provided on the actuator 41. As a result, the volume of the pump chamber 1c increases, and the pressure in the pump chamber 1c drops. Accordingly, the first check valve 4 opens, and the fluid flows from the outside into the pump chamber 1c.

Since one of the discharge valves of the actuator is covered with a cylindrical member to form a check valve as described above, the number of components can be reduced, and an air pump with a simpler structure can be provided.

Although the EPAM (1) shown in FIG. 2A is used for the actuator 41 in this embodiment, the same effects as the above can be achieved by employing the EPAM (2) shown in FIG. 2B.

[Seventh Embodiment]

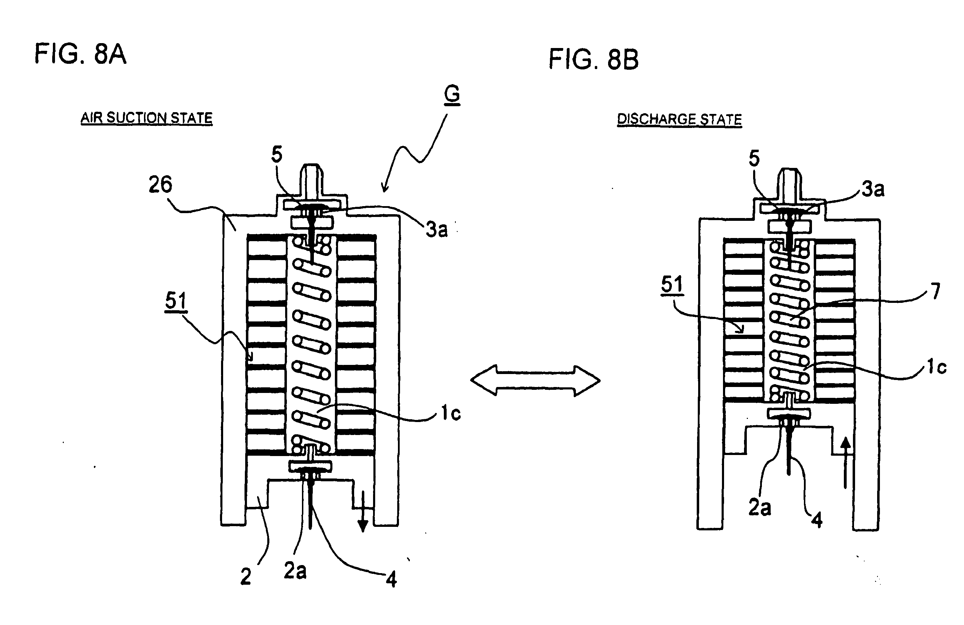

Referring now to FIGS. 8A and 8B, the structure of an air pump in accordance with a seventh embodiment is described.

An air pump G in accordance with the seventh embodiment differs from the air pump A of the first embodiment in that a housing member 26 covers and protects an actuator 51 so as to provide a guiding function for expansion and contraction. In the following, explanation is focused on the differences in the structure, functions, and effects between the first and seventh embodiments, and explanation of the same components as those of the first embodiment is omitted.

As shown in FIG. 8A, in a case where the voltage to be applied to the actuator 51 is OFF, the actuator 51 stands still at such a location that the volume of the pump chamber 1c becomes the largest. In this situation, the pressure is almost the same between the inside and the outside of the pump chamber 1c. Accordingly, the fluid introduced through the suction unit 2a flows into the pump chamber 1c through the gap between the check valve 4 and the housing member 2.

After that, when the voltage to be applied to the actuator 51 is turned ON, the actuator 51 contracts in its longitudinal direction, and the volume of the pump chamber 1c becomes smaller, as shown in FIG. 8B. At this point, the first check valve 4 closes, as the pressure inside the pump chamber 1c increases. Accordingly, the fluid flowing through the suction unit 2a is shut off. As the pump chamber 1c is compressed, the pressure inside the pump chamber 1c further increases and the second check valve 5 opens. As a result, the fluid is discharged to the outside through the discharge unit 3a.

When the voltage to be applied to the actuator 51 is again turned OFF, the actuator 51 expands in its longitudinal direction by virtue of the restoring force of the flexible member 7 provided in the actuator 1. As a result, the volume of the pump chamber 1c increases, and the pressure in the pump chamber 1c drops. Accordingly, the fluid flows from the outside into the pump chamber 1c.

In this structure, the housing member 26 covers the outer surface of the actuator 51, thereby protecting the elastomer or polymer members 1a and providing a guiding function for the actuator 51 to expand and contract.

With its reciprocating movement (expansion and contraction), the actuator 51 repeatedly switches between the states shown in FIGS. 8A and 8B, thereby changing the volume of the pump chamber 1c and the pressure in the pump chamber 1c of the actuator 51. Thus, an air pump with a simple structure can be provided, without a power transmission device such as a motor or a gear.

With the above described air pump G in accordance with the seventh embodiment, a complicated mechanism- such as a conventional motor or clutch is not necessary, and a component for converting the direction of movement from rotating movement to reciprocating movement is not required either. Accordingly, an air pump with a simple structure can be provided. Also, such an air pump is smaller and lighter than a conventional air pump with a motor.

Since the housing member 26 serves to protect the actuator 51 and serves to guide the actuator 51 at the time of expansion and contraction, an air pump with stable pumping performance can be provided.

Although the EPAM (1) shown in FIG. 2A is used for the actuator 51 in this embodiment, the same effects as the above can be achieved by employing the EPAM (2) shown in FIG. 2B.

[Eighth Embodiment]

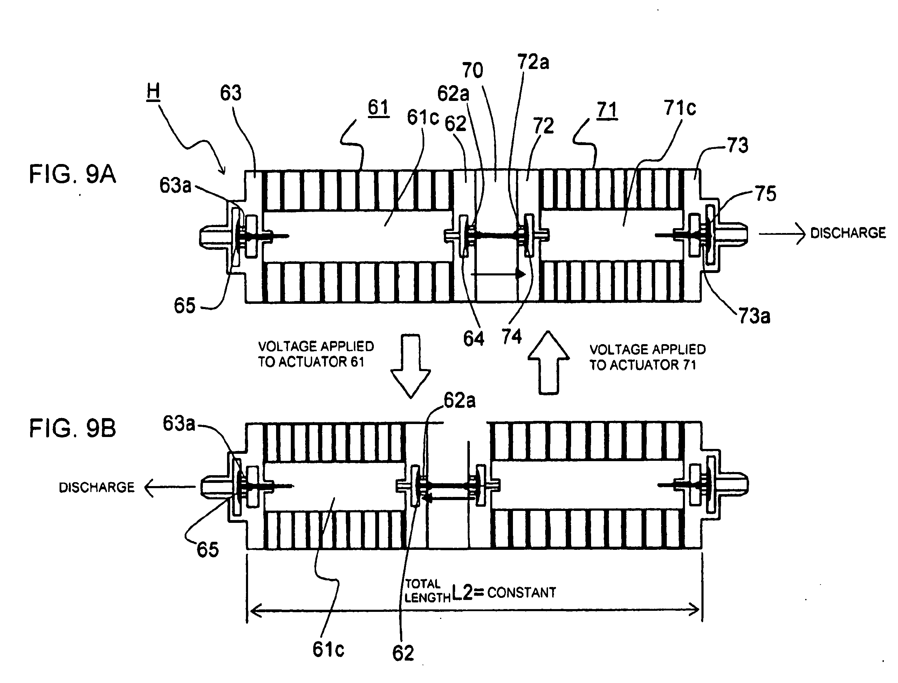

Referring now to FIGS. 9A and 9B, the structure of an air pump in accordance with an eighth embodiment is described.

In an air pump H in accordance with the eighth embodiment, two actuators are connected in series, and suction and discharge are conducted through the connecting parts between the two actuators. With this arrangement, the air pump H can expand and contract without a spring.

In a case where the voltage to be applied to actuators 61 and 71 is OFF, the actuators 61 and 71 are fixed to a casing (not shown) to stand still and maintain a total length L2. In this situation, the pressure is almost the same between the inside and the outside of pump chambers 61c and 71c. Accordingly, the fluid introduced from suction units 62a and 72a through an inlet 70 flows into the pump chambers 61c and 71c through the gap between first check valves 64 and 74 and housing members 62 and 72.

After that, when the voltage to be applied to the actuator 61 is turned ON, the actuator 61 contracts in its transverse direction, and the volume of the pump chamber 61c becomes smaller, as shown in FIG. 9B. At this point, the first check valve 64 closes, as the pressure inside the pump chamber 61c increases. Accordingly, the fluid flowing from the suction unit 62a is shut off. As the pump chamber 61c is compressed, the pressure inside the pump chamber 61c further increases and the second check valve 65 opens. As a result, the fluid is discharged to the outside through the discharge unit 63a.

After that, when the voltage to be applied to the actuator 61 is turned OFF and the voltage to be applied to the actuator 71 is turned ON, the actuator 71 contracts while the actuator 61 expands in its transverse direction. As a result, the volume of the pump chamber 61c increases, and the pressure in the pump chamber 61c drops. Accordingly, the second check valve 65 closes, and the fluid flowing through the discharge unit 63a is shut off. As the pump chamber 61c is compressed, the pressure in the pump chamber 61c further drops. As a result, the first check valve 64 opens, and the fluid is introduced from the suction unit 62a into the pump chamber 61c via the inlet 70.

Meanwhile, the actuator 71 contracts in its transverse direction, and the volume of the pump chamber 71c becomes smaller. At this point, the pressure in the pump chamber 71c increases, and the first check valve 74 closes to shut off the fluid flowing from the suction unit 72a. As the pump chamber 71c is compressed, the pressure in the pump chamber 71c further increases. As a result, the second check valve 75 opens, and the fluid is discharged to the outside through the discharge unit 73a.

As described above, the actuators 61 and 71 are connected in series, and the total length is made constant. Thus, the force of one of the actuators at the time of compression can be used as the restoring force for the other compressed actuator.

Through the reciprocating movement (expansion and contraction), the actuators 61 and 71 repeatedly switch between the states shown in FIGS. 9A and 9B, thereby changing the volume of each of the pump chambers 61c and 71c and the pressure in each of the pump chambers 61c and 71c, without an elastic member such as a spring. Thus, an air pump with a simple structure can be provided, without a power transmission device such as a motor or a gear.

With the above described air pump H in accordance with the eighth embodiment, a complicated mechanism such as a conventional motor or a clutch is not necessary, and a component for converting the direction of movement from rotating movement to reciprocating movement is not required either. Accordingly, an air pump with a simple structure can be provided. Also, such an air pump is smaller and lighter than a conventional air pump with a motor.

With this air pump H, there is no driving noise of a motor. Accordingly, the driving noise of the air pump can be reduced, since there are no components for converting the direction of movement. Furthermore, the air pump H can operate with a lower current than a current required for driving a motor.

Compared with the case where the disk-like electrostriction polymer actuator of the prior art is employed as a diaphragm, the volume change of the pump chamber can be easily made greater.

Although the EPAM (1) shown in FIG. 2A is used for the actuators 61 and 71 in the this embodiment, the same effects as the above can be achieved by employing the EPAM (2) shown in FIG. 2B.

[Ninth Embodiment]

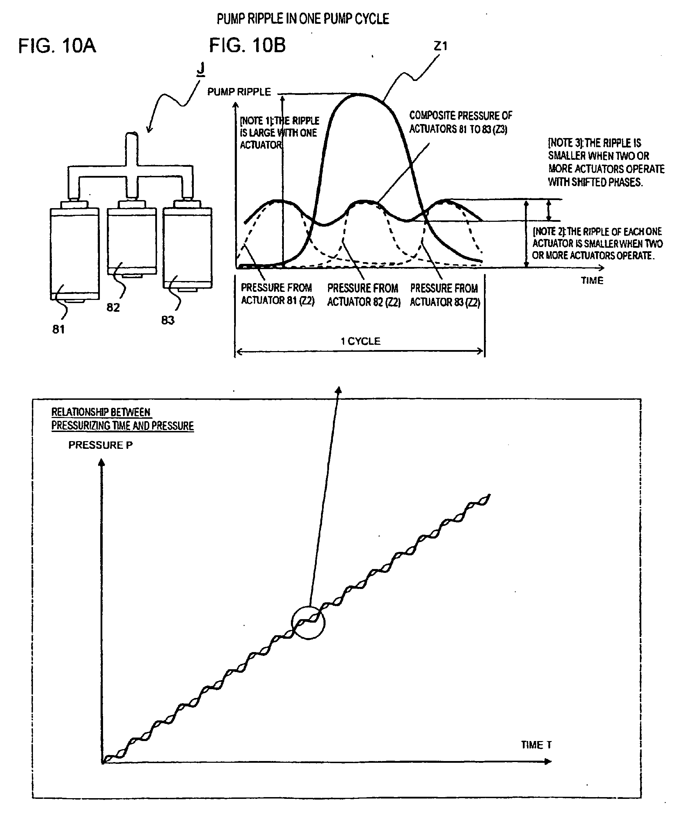

Referring now to FIGS. 10A and 10B, the structure of a pump system in accordance with a ninth embodiment that employs a plurality of the above described air pumps is described.

In a case where a single pump using EPAM for the actuator operates, the pressure variation (a ripple) caused when the EPAM expands and contracts becomes greater, as indicated by the curve Z1 in FIG. 10B.

In a case where the above pump is used for monitoring blood pressure according to a pressurizing measurement method, if the ripple is greater than a certain value, the ripple is sensed as the pulse wave of the human body, and might adversely affect the accuracy in blood pressure measurement.

Therefore, an air pump J in accordance with the ninth embodiment has a plurality of actuators connected in parallel, and the same flow rate of fluid as that discharged by a single pump in one cycle is discharged by a plurality of pumps in the same one cycle. In this structure, the flow rate per pump is reduced, and the ripple can be made smaller accordingly.

The air pump J has three actuators 81, 82, and 83 connected in parallel, and the discharge units of the respective actuators are linked so as to serve as a single pump. The actuators 81, 82, and 83 are formed with EPAM. To discharge the same flow rate of fluid as that discharged by a pump with a single actuator, the flow rate to be discharged by each actuator should be approximately a third of it. More specifically, the air pump J is a pump system that includes two or more air pumps having the discharge units connected to one another. The phases of the voltages to be applied to the actuators of the air pumps are varied. By doing so, the ripples Z2 generated by the air pumps can be made smaller than the ripple Z1.

More preferably, the timing (the phase) of suction and discharge of each actuator is shifted $2\pi/n$ from that of each neighboring actuator, where n is the number of air pumps. In a case where the number of air pumps is three, for example, the phases should be shifted $2\pi/3$ from one another. By doing so, the ripple caused by the three actuators can be made even smaller as indicated by the curve Z3.

It should be noted that the number of actuators is not limited to three, but may be any number that is two orgreater. The phases of the actuators shouldbe shifted $2\pi/n$ (n being an integer of 2 or greater) from one another.

In this manner, the actuators are connected in parallel, and the same flow rate of fluid as that discharged by a single pump is discharged by a plurality of pumps. The operating timings of the actuators are shifted from one another, so that the ripple caused by the discharged fluid can be made smaller. As a result, the pump system can be used for a blood pressure monitor, so as to perform measurement with high precision.

With the above described air pump J in accordance with the ninth embodiment, a complicated mechanism such as a conventional motor or clutch is not necessary, and a component for converting the direction of movement from rotating movement to reciprocating movement is not required either. Accordingly, an air pump with a simple structure can be provided. Also, such an air pump is smaller and lighter than a conventional air pump with a motor.

With this air pump J, there is no driving noise of a motor. Accordingly, the driving noise of the air pump can be reduced, since there are no components for converting the direction of movement. Furthermore, the air pump J can operate with a lower current than a current required for driving a motor.

Compared with the case where the disk-like electrostriction polymer actuator of the prior art is employed as a diaphragm, the volume change of the pump chamber can be easily made greater.

Although the EPAM (1) shown in FIG. 2A is used for the actuators 81, 82, and 83 in the this embodiment, the same effects as the above can be achieved by employing the EPAM (2) shown in FIG. 2B.

[Tenth Embodiment]

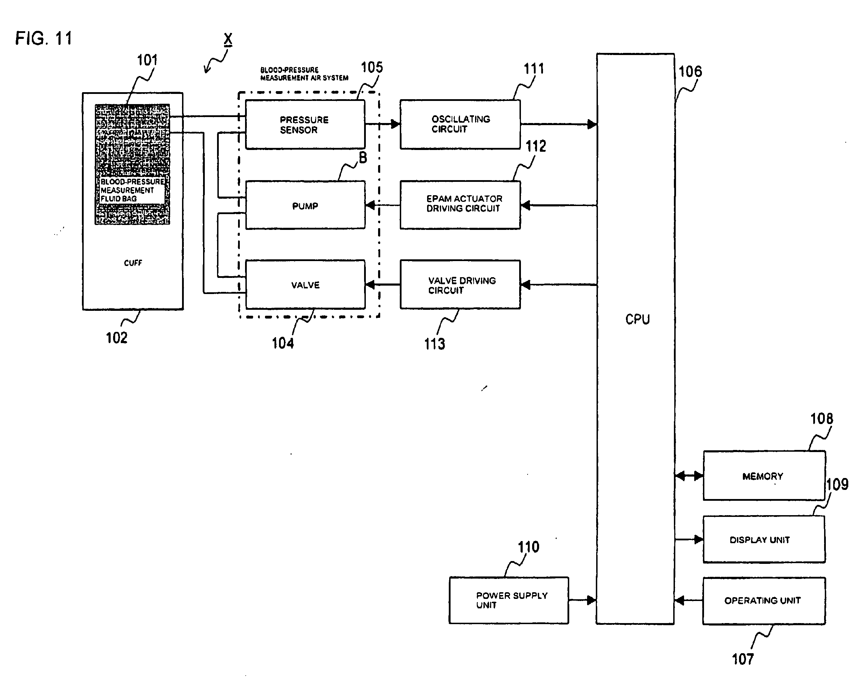

A tenth embodiment of the present invention concerns an electronic blood pressure monitor in which any of the above air pumps can be suitably employed. FIG. 11 is a block diagram illustrating the hardware structure of the electronic blood pressure monitor. Although the air pump A of the first embodiment is employed in this embodiment, it is of course possible to employ any of the air pumps B through J of the second through ninth embodiments.

(Structure of Blood Pressure Monitor)

An electronic blood pressure monitor X includes: a fluid bag 101 that is wound around an upper arm (a living body) at the time of blood pressure measurement; a pressing and fixing cuff 102 that externally presses and fixes the fluid bag 101; the air pump A that introduces fluid into the fluid bag 101, which is filled with fluid such as air, and pressurizes the fluid bag 101; a valve 104 that discharges fluid from the fluid bag 101; a pressure sensor 105 that senses the inner pressure of the fluid bag 101; a CPU 106 that serves as an operating unit for performing an operation for blood pressure measurement according to a stored program, based on the sensed inner pressure; an operating unit 107 that sets the measurement time and starts the measurement; a memory 108 that stores data such as setting data, operating data, and measurement results; a display unit 109 that displays data such as setting conditions and measurement results; and a power supply unit 110 that supplies power to each component.

The CPU 106 also detects the pressure inside the fluid bag 101, based on a signal that is output from the pressure sensor 105 and is converted by an oscillating circuit 111. If pressurizing is required, the air pump A is driven by the CPU 106 using the driving circuit 112 of the actuator 1 with the EPAM, so as to increase the pressure in the fluid bag 101. Meanwhile, if depressurizing is required, the valve 104 is opened by a valve driving circuit 113, so as to reduce the pressure in the fluid bag 101.

(Basic Operation of Blood Pressure Monitor)

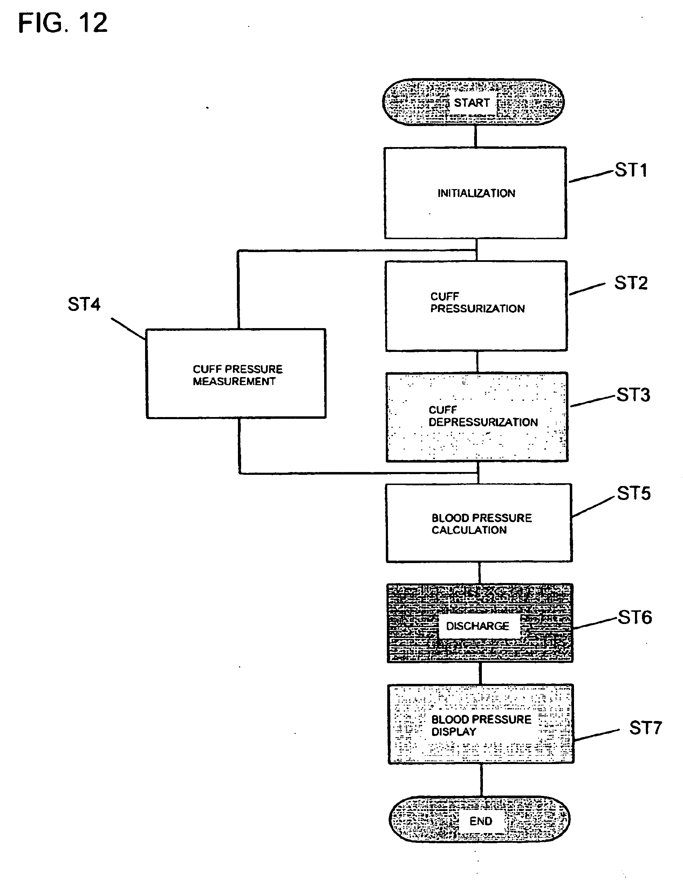

FIG. 12 is a flowchart of the basic operation of an electronic blood pressure to which the present invention is suitably applied. Although measurement is carried out with respect to an upper arm of a human body in the following description, it is also possible to use the electronic blood pressure monitor on living bodies other than human bodies, and to perform measurement on a wrist or an ankle that is a part of a living body.

First, the cuff is wound around an upper arm (a living body), and the power supply is turned ON. As the operation stars, initializing is performed to reset each setting state of the electronic blood pressure monitor X to the initial state (step ST1).

The fluidbag 101 wound around the upper arm (a living body) is pressurized to a predetermined pressure by the air pump A (step ST2). At the same time, the signal indicating the pressure change of the fluid bag 101 detected by the pressure sensor 105 is transmitted to the CPU 106 via the oscillating circuit 111, and measurement is started according to the signal (step ST4).

After the pressurization, the pressure in the fluid bag 101 is gradually reduced as the valve 104 is opened (step ST3) . At the same time, the signal indicating the pressure change of the fluid bag 101 detected by the pressure sensor 105 is transmitted to the CPU 106 via the oscillating circuit 111 . The CPU 106 then calculates the systolicbloodpressure, thediastolicbloodpressure, and the pulse rate (step ST5).

After the measurement is completed, the air in the fluid bag 101 pressing the upper arm is discharged through the valve 104, and the upper arm is released from the pressure (step ST6).

The calculated blood pressure value or the like is displayed on the display unit 109 (step ST7), and the measurement of one cycle is ended.

[Eleventh Embodiment]

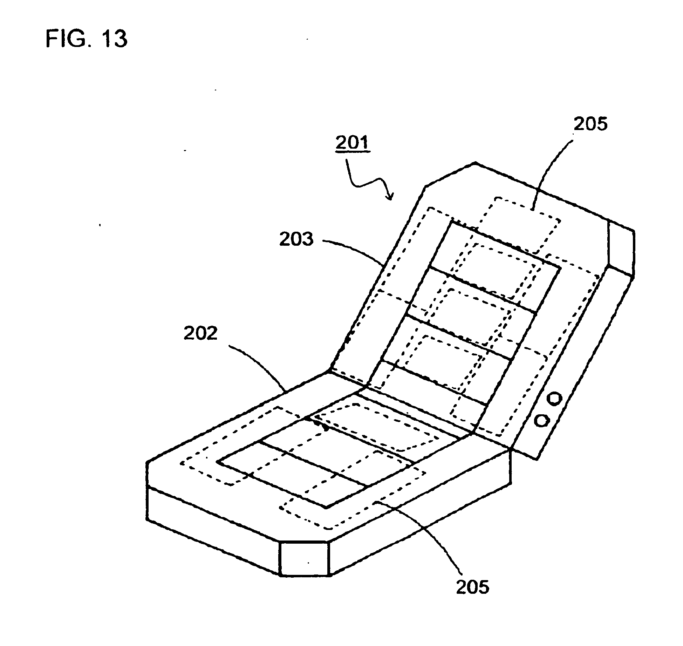

An eleventh embodiment of the present invention concerns a massager to which any of the above air pumps can be suitably applied. FIG. 13 is an external perspective view illustrating the fundamental structure of the massager in accordance with the eleventh embodiment. Although the air pump A of the first embodiment is employed in this embodiment, it is of course possible to employ any of the air pumps B through J of the second through ninth embodiments.

(Structure of Massager)

A massager 201 is formed with a seat 202 and a back 203 in appearance, like a regular legless chair. Inside the seat 202 and the back 203, a plurality of air bags 205 that expand and contract with air charge and discharge are provided, and the air pump A (not shown) is connected to each of the air bags 205. The massager 201 further includes air controlling means (not shown) that controls air charge and discharge to and from the air bags 205.

In this embodiment, each of the air bags 205 has a rectangular shape. Three of the air bags 205 are provided to the seat 202, and eight of the air bags 205 are provided to the back 203. It should be noted that the shape of each air bag 205 is not necessarily rectangular, but each air bag 205 may have a circular, triangular, oval shape or the like. Also, the number of air bags 205 may be increased or decreased according to the size, shape and the like of each air bag 205. In this massager 201, air is introduced into or discharged from the air bags 205 by the air controlling means, so that the air bags 205 expand and contract to massage a human body.

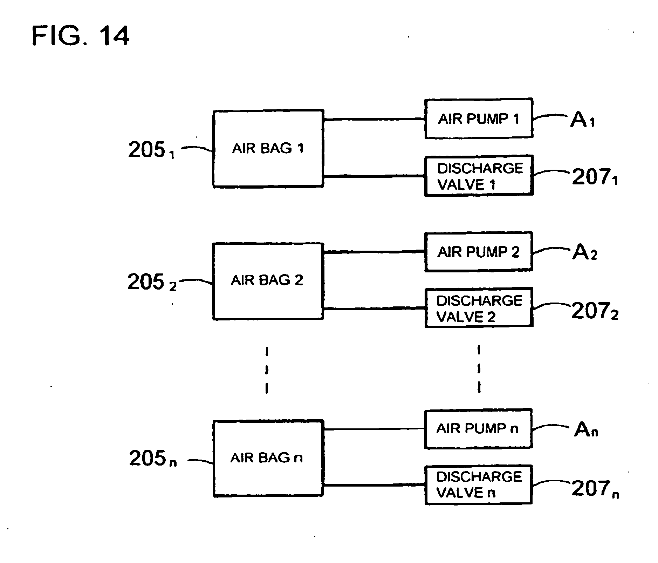

In addition to the above fundamental structure, the massager 201 may have the following structure. FIG. 14 is a block diagram of the main components of the massager. The structure shown in FIG. 14 includes air pumps A and discharge valves 207, with the air controlling means being provided for each air bag. In FIG. 14, n of air bags $205_1$, $205_2$, ..., $205_n$ are provided over the seat 202 and the back 203 of the massager 201, and the air pumps $A_1$, $A_2$, ..., $A_n$ and the discharge valves $207_1$, $207_2$, ..., $207_n$ are provided for the respective air bags $205_1$, $205_2$, ..., $205_n$. The air pumps $A_1$, $A_2$, ..., $A_n$ and the discharge valves $207_1$, $207_2$, ..., $207_n$ form the air controlling means.

In FIG. 14, the air pumps $A_1$, $A_2$, ..., $A_n$ and the discharge valves $207_1$, $207_2$, ..., $207_n$ correspond to the respective air bags $205_1$, $205_2$, ..., $205_n$. Therefore, in a case where the air bag $205_1$ is to expand, for example, the air pump $A_1$ is actuated to introduce air into the air bag $205_1$, with the discharge valve $207_1$ being closed. When the inner pressure of the air bag $205_1$ reaches a predetermined pressure, the operation of the air pump $A_1$ is suspended. In a case where the air bag $205_1$ is to contract, the discharge valve $207_1$ is opened to discharge the air from the air bag $205_1$.

(Massager with another Structure)

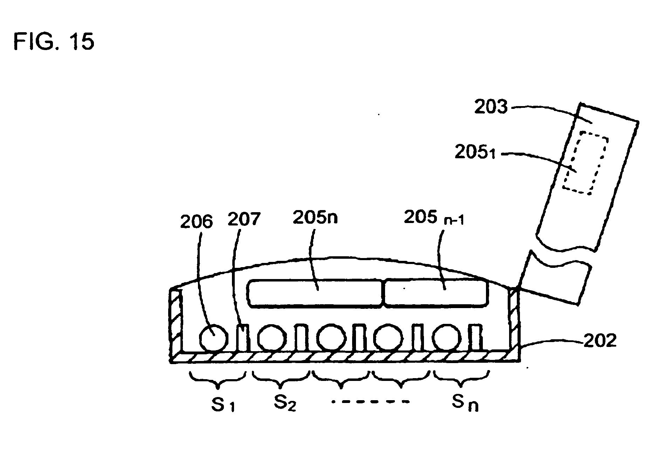

FIG. 15 is a cross-sectional view illustrating the main components of the massager 201 with another structure. In this massager 201, the air pumps A ($A_1$, $A_2$, ..., $A_n$) and the discharge valves 207 ($207_1$, $207_2$, ..., $207_n$) corresponding to n of air bags $205_1$, $205_2$, ... , $205_n$ are provided in the seat 202. More specifically, the air pump $A_1$ and the discharge valve $207_1$ corresponding to the air bag $205_1$ form an $S_1$ set, and the air pump $A_n$ and the discharge valve $207_n$ corresponding to the air bag $205_n$ form an $S_n$ set. The sets $S_1$ through $S_n$ are sequentially arranged. In this case, the air pumps A and the discharge valves 207 that are the main source of noise are far apart from the upper portion of the back 203 to which the face of a human face (ear) is set close. Accordingly, to the ear of each user, the air pumps A are less noisy.

Since the air pumps A and the discharge valves 207 are located close to the respective air bags 205, the flow path (a tube, for example) connecting each air bag 205 to each corresponding air pump A can be short. Accordingly, the air pressure in the flow path can be restrained from decreasing, and the air can be efficiently introduced into each air bag 205 from each corresponding air pump A. Thus, excellent responsiveness can be achieved in expansion and contraction of the air bags 205.

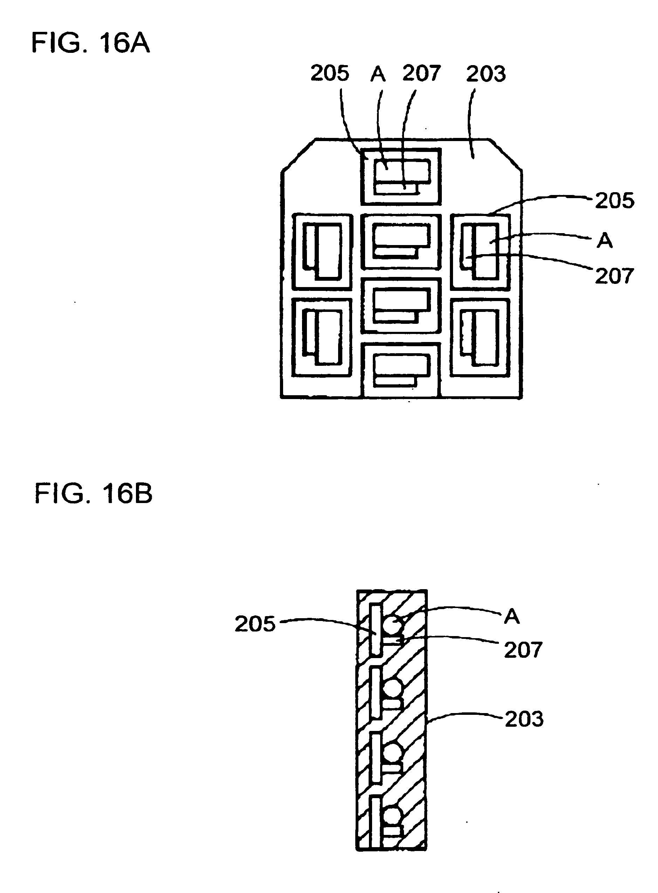

FIGS. 16A and 16B illustrate a specific example of the arrangement of the air pumps A and the discharge valves 207. FIG. 16A is a rear view of the back 203, and FIG. 16B is a vertical cross-sectional view of the back 203, taken along the center line and seen from the right side. Here, the air pumps A and the discharge valves 207 are located on the opposite side (on the rear surface side) from the body contact surface side of each air bag 205. Each air pump A is placed on the upper side of the rear region of the corresponding air bag 205, and the corresponding discharge valve 207 is placed on the lower side of the rear region. This arrangement is made for all the air bags 205 provided in the back 203. It is of course possible to make the same arrangement as the above in the seat 202. As those components are arranged in the depth direction of the massager 201 as described above, a large body contact surface of the back 203 can be maintained, and a higher degree of freedom can be allowed for the arrangement of the air bags 205 with respect to the body contact surface.

In this embodiment, air pumps that do not require a complicated mechanism such as a conventional motor or clutch and do not require a component for converting the direction of movement from rotational movement to reciprocating movement are used for expansion and contraction of the air bags provided in a massager. Accordingly, a massager with a simpler structure can be provided. Furthermore, such a massager can be made smaller and lighter than a massager using air pumps with motors.

Also, as the driving noise of motors is eliminated and any component for converting the direction of movement is not employed, the noise of the air pumps can be reduced at the time of operation. Accordingly, users feel less discomfort during the use of the massager. In addition to that, the massager can operate with lower current than the current required for driving motors.

The present invention is not limited to the above embodiments, and various modifications and combinations can be made, without departing from the scope of protection defined by the subject-matter of the appended patent claims.

The present invention can be applied not only to a chair-type air massager that is formed with the above described seat and back, but also to an air massager that massages only a leg or a hand.

**Claims**

1. An air pump comprising:

   a first hollow actuator (22, 23; 61, 71) that has elastomer or polymer members that can expand and contract in response to voltage application, and electrodes (24, 25) for applying voltage to the elastomer or polymer members;
   a suction unit (2a; 62a, 72a) that sucks fluid into the hollow actuator and a discharge unit (3a; 63a, 73a) that discharges fluid to the outside of the hollow actuator upon expansion or contraction of said first actuator, **characterized by**
   a second hollow actuator (23, 22; 71, 61) that has elastomer or polymer members that can expand and contract in response to voltage application, and electrodes (25, 24) for applying voltage to the elastomer or polymer members, said first and second actuators (22, 23; 61, 71) being arranged such that a contraction of said first actuator causes an expansion of said second actuator and a contraction of said second actuator causes an expansion of said first actuator.

2. An air pump according to claim 1, comprising:

   a pump chamber surrounding said first and second actuators (22, 23), wherein:

   said first actuator (22) is arranged to reduce the volume of the pump chamber by voltage application, and said second actuator (23) is arranged to increase the volume of the pump chamber by voltage application; the volume of the pump chamber being varied by alternately applying voltage to the first actuator (22) and the second actuator (23), thereby discharging pressurized fluid to the outside.

3. An air pump according to claim 1, wherein:

   said first and second actuators (61, 71) are connected in series, suction and discharge being conducted through the connecting parts; and
   the total length of said connected first and second actuators (61, 71) in the direction of expansion and contraction is constant.

4. An air pump according to any of the preceding claims, wherein said first and second actuators (22, 23; 61, 71) have a varied extent of expansion and contraction depending on the magnitude of an applied voltage.

5. An air pump according to any of the preceding claims, wherein voltage to be applied to said first and second actuators (22, 23; 61, 71) or frequency of the applied voltage are controlled to control the discharge flow rate and discharge pressure of fluid to be discharged.

6. A pump system comprising:

   a plurality of air pumps according to any of the preceding claims, with discharge units of the air pumps being connected to one another,
   the phases of voltages to be applied to actuators of the air pumps being shifted from one another.

7. A pump system according to claim 6, wherein the phases are shifted $2\pi/n$ from one another, with n being the number of air pumps.

8. An electronic blood pressure monitor comprising:

   a fluid bag that is filled with fluid such as air and is wound around a living body;
   a cuff that externally fixes the fluid bag;
   the air pump according to any of the claims 1 to 5 that introduces fluid into the fluid bag and pressurizes the fluid bag;
   a pressure sensor that detects the inner pressure of the fluid bag; and
   operating means that performs an operation for blood pressure measurement based on the detected inner pressure.

**9.** An electronic blood pressure monitor comprising:

a fluid bag that is filled with fluid such as air and is wound around a living body;
a cuff that externally fixes the fluid bag;
the pump system according to claim 6 or 7 that introduces fluid into the fluid bag and pressurizes the fluid bag;
a pressure sensor that detects the inner pressure of the fluid bag; and
operating means that performs an operation for blood pressure measurement based on the detected inner pressure.

**Patentansprüche**

**1.** Luftpumpe, welche aufweist:

einen ersten hohlen Aktuator (22, 23; 61, 71), der Elastomer- oder Polymerelemente aufweist, die sich ansprechend auf ein Anlegen von Spannung ausdehnen und zusammenziehen können, und Elektroden (24, 25) zum Anlegen von Spannung an die Elastomer- oder Polymerelemente;
eine Saugeinheit (2a; 62a, 72a), die Fluid in den hohlen Aktuator saugt, und eine Abgabeeinheit (3a; 63a, 73a), die Fluid aus dem hohlen Aktuator abgibt, mit Ausdehnung oder Kontraktion des ersten Aktuators, **gekennzeichnet durch**
einen zweiten hohlen Aktuator (23, 22; 71, 61), der Elastomer- oder Polymerelemente aufweist, die sich ansprechend auf ein Anlegen von Spannung ausdehnen und zusammenziehen können, und Elektroden (25, 24) zum Anlegen von Spannung an die Elastomer- oder Polymerelemente, wobei der erste und der zweite Aktuator (22, 23; 61, 71) so eingerichtet sind, dass ein Zusammenziehen des ersten Aktuators ein Ausdehnen des zweiten Aktuators bewirkt und ein Zusammenziehen des zweiten Aktuators ein Ausdehnen des ersten Aktuators bewirkt.

**2.** Luftpumpe nach Anspruch 1, welche aufweist:

eine den ersten und den zweiten Aktuator (22, 23) umschließende Pumpenkammer, wobei:

der erste Aktuator (22) so eingerichtet ist, dass er das Volumen der Pumpenkammer durch Anlegen von Spannung vermindert, und der zweite Aktuator (23) so eingerichtet ist, dass er das Volumen der Pumpenkammer durch Anlegen von Spannung erhöht;

wobei das Volumen der Pumpenkammer durch abwechselndes Anlegen von Spannung an den ersten Aktuator (22) und den zweiten Aktuator (23) verändert wird, womit unter Druck stehendes Fluid nach außen abgegeben wird.

**3.** Luftpumpe nach Anspruch 1, wobei
der erste und der zweite Aktuator (61, 71) in Reihe verbunden sind, wobei ein Ansaugen und Abgeben über die Verbindungsteile geführt wird; und
die Gesamtlänge aus verbundenem ersten und zweiten Aktuatoren (61, 71) in Richtung des Ausdehnens und Zusammenziehens konstant ist.

**4.** Luftpumpe nach irgendeinem der vorstehenden Ansprüche, wobei der erste und der zweite Aktuator (22, 23; 61, 71) ein verändertes Ausmaß an Ausdehnung und Zusammenziehen, abhängig von der Größe einer angelegten Spannung, haben.

**5.** Luftpumpe nach irgendeinem der vorstehenden Ansprüche, wobei die an dem ersten und dem zweiten Aktuator (22, 23; 61, 71) anzulegende Spannung oder die Frequenz der angelegten Spannung so gesteuert werden, dass die Abgabeflussgeschwindigkeit und der Abgabedruck des abzugebenden Fluids gesteuert werden.

**6.** Pumpensystem, welches aufweist:

eine Anzahl von Luftpumpen nach irgendeinem der vorstehenden Ansprüche, wobei Abgabeeinheiten der Luftpumpen miteinander verbunden sind,

wobei die Phasen von an Aktuatoren der Luftpumpen anzulegenden Spannungen gegeneinander verschoben sind.

**7.** Pumpensystem nach Anspruch 6, wobei die Phasen um $2\pi/n$ gegeneinander verschoben sind, wobei n die Anzahl von Luftpumpen ist.

**8.** Blutdruckmessgerät, welches aufweist:

einen Fluidsack, der mit einem Fluid, wie etwa Luft, gefüllt und um einen lebenden Körper gewickelt ist;
eine Manschette, die den Fluidsack außen festlegt;
die Luftpumpe nach irgendeinem der Ansprüche 1 bis 5, die Fluid in den Fluidsack einführt und das Fluid unter Druck setzt;
einen Drucksensor, der den Innendruck des Fluidsacks feststellt; und
Betriebsmittel, die einen Betrieb zur Blutdruckmessung beruhend auf dem festgestellten Innendruck durchführen.

**9.** Elektronisches Blutdruckmessgerät, welches aufweist:

einen Fluidsack, der mit einem Fluid, wie etwa Luft, gefüllt und um einen lebenden Körper gewickelt ist;
eine Manschette, die den Fluidsack außen festlegt;
das Pumpensystem nach Anspruch 6 oder 7, das Fluid in den Fluidsack einführt und den Fluidsack unter Druck setzt;
einen Drucksensor, der den Innendruck des Fluidsacks feststellt; und
Betriebsmittel, die einen Betrieb zur Blutdruckmessung beruhend auf dem festgestellten Innendruck durchführen.

**Revendications**

**1.** Pompe à air comprenant :

♦ un premier actionneur creux (22, 23 ; 61, 71) comportant des éléments élastomères ou polymères qui peuvent se dilater et se contracter en réponse à l'application d'une tension, et des électrodes (24, 25) destinées à appliquer cette tension aux éléments élastomères ou polymères ;
♦ une unité d'aspiration (2a ; 62a, 72a) aspirant le fluide dans l'actionneur creux et une unité d'évacuation (3a ; 63a, 73a) évacuant le fluide hors de l'actionneur creux lors de la dilatation ou de la contraction dudit premier actionneur, **caractérisée par** un second actionneur creux (23, 22 ; 71, 61) qui comporte des éléments élastomères ou polymères qui peuvent se dilater et se contracter en réponse à l'application d'une tension, et des électrodes (25, 24) destinées à appliquer une tension aux éléments élastomères ou polymères, lesdits premier et second actionneurs (22, 23 ; 61, 71) étant agencés de telle sorte qu'une contraction dudit premier actionneur entraîne une dilatation dudit second actionneur, et qu'une contraction dudit second actionneur entraîne une dilatation dudit premier actionneur.

**2.** Pompe à air selon la revendication 1, comprenant :

♦ une chambre de pompe entourant lesdits premier et second actionneurs (22, 23), dans laquelle :

♦ ledit premier actionneur (22) est agencé de façon à réduire le volume de la chambre de pompe par application d'une tension, et ledit second actionneur (23) est agencé de façon à augmenter le volume de la chambre de pompe par application d'une tension ;
♦ le volume de la chambre de pompe varie en appliquant alternativement une tension au premier actionneur (22) et au second actionneur (23), en évacuant ainsi le fluide sous pression vers l'extérieur.

**3.** Pompe à air selon la revendication 1, dans laquelle :

♦ lesdits premier et second actionneurs (61, 71) sont raccordés en série, l'aspiration et l'évacuation s'effectuant à travers les parties de raccordement ; et
♦ la longueur totale desdits premier et second actionneurs (61, 71) raccordés, dans le sens de la dilatation et de la contraction, est constante.

**4.** Pompe à air selon l'une quelconque des revendications précédentes, dans laquelle lesdits premier et second actionneurs (22, 23 ; 61, 71) présentent une proportion variable de dilatation et de contraction selon l'amplitude de la tension appliquée.

5. Pompe à air selon l'une quelconque des revendications précédentes, dans laquelle la tension à appliquer auxdits premier et second actionneurs (22, 23 ; 61, 71) ou la fréquence de la tension appliquée sont contrôlées pour commander le débit d'évacuation et la pression d'évacuation du fluide à évacuer.

6. Système de pompe comprenant une pluralité de pompes à air selon l'une quelconque des revendications précédentes, les unités d'évacuation des pompes à air étant raccordées l'une à l'autre, les phases des tensions à appliquer aux actionneurs des pompes à air étant décalées de l'une à l'autre.

7. Système de pompe selon la revendication 6, dans lequel les phases sont décalées de $2\pi/n$, n étant le nombre de pompes à air.

8. Appareil électronique de surveillance de pression sanguine comprenant :

   ♦ une poche de fluide qui est remplie d'un fluide tel de l'air et est enroulée autour d'un corps vivant ;
   ♦ un bracelet fixant à l'extérieur la poche de fluide ;
   ♦ la pompe à air selon l'une quelconque des revendications 1 à 5 introduisant le fluide dans la poche de fluide et pressurisant la poche de fluide ;
   ♦ un capteur de pression détectant la pression intérieure de la poche de fluide ; et
   ♦ des moyens d'actionnement exécutant une opération de mesure de pression sanguine en se basant sur la pression intérieure détectée.

9. Appareil électronique de surveillance de pression sanguine comprenant :

   ♦ une poche de fluide qui est remplie d'un fluide tel de l'air et est enroulée autour d'un corps vivant ;
   ♦ un bracelet qui fixe à l'extérieur la poche de fluide ;
   ♦ le système de pompe selon la revendication 6 ou 7 qui introduit le fluide dans la poche de fluide et pressurise la poche de fluide ;
   ♦ un capteur de pression détectant la pression intérieure de la poche de fluide ; et
   ♦ des moyens d'actionnement exécutant une opération de mesure de pression sanguine en se basant sur la pression intérieure détectée.

FIG. 1A

AIR SUCTION STATE

FIG. 1B

DISCHARGE STATE

## FIG. 2A

EPAM (1) CONTRACTS WITH VOLTAGE APPLICATION

## FIG. 2B

EPAM (2) EXPANDS WITH VOLTAGE APPLICATION

FIG. 3A

FIG. 3B

# FIG. 4A

AIR SUCTION STATE

# FIG. 4B

DISCHARGE STATE

EPAM (1) CONTRACTS WITH VOLTAGE APPLICATION

RESTORED BY RESTORING FORCE OF FLEXIBLE MEMBERS

FIG. 5B

DISCHARGE STATE

EPAM (1) CONTRACTS WITH VOLTAGE APPLICATION

RESTORED BY RESTORING FORCE OF SPRING

FIG. 5A

AIR SUCTION STATE

FIG. 6A

COMPRESSED BY APPLYING
VOLTAGE TO EPAM (1)

EXPANDED BY APPLYING
VOLTAGE TO EPAM (2)

FIG. 6B

# FIG. 7A

# FIG. 7B

# FIG. 7C

SECTION A-A

EP 1 643 126 B1

FIG. 8B
DISCHARGE STATE

FIG. 8A
AIR SUCTION STATE

FIG. 9A

FIG. 9B

PUMP RIPPLE IN ONE PUMP CYCLE

FIG. 10A          FIG. 10B                                    Z1

FIG. 10A

_J_

FIG. 10B

PUMP RIPPLE

[NOTE 1]: THE RIPPLE IS LARGE WITH ONE ACTUATOR

COMPOSITE PRESSURE OF ACTUATORS 81 TO 83 (Z3)

[NOTE 3]: THE RIPPLE IS SMALLER WHEN TWO OR MORE ACTUATORS OPERATE WITH SHIFTED PHASES.

[NOTE 2]: THE RIPPLE OF EACH ONE ACTUATOR IS SMALLER WHEN TWO OR MORE ACTUATORS OPERATE.

TIME

PRESSURE FROM ACTUATOR 81 (Z2)    PRESSURE FROM ACTUATOR 82 (Z2)    PRESSURE FROM ACTUATOR 83 (Z2)

1 CYCLE

82      83      81

RELATIONSHIP BETWEEN PRESSURIZING TIME AND PRESSURE

PRESSURE P

TIME T

FIG. 11

X

BLOOD-PRESSURE
MEASUREMENT AIR SYSTEM    105

101

PRESSURE
SENSOR

BLOOD-PRESSURE
MEASUREMENT
FLUID BAG

CUFF

102

B

PUMP

VALVE

104

OSCILLATING
CIRCUIT        111

EPAM ACTUATOR
DRIVING CIRCUIT    112

VALVE DRIVING
CIRCUIT        113

CPU            106

MEMORY         108

DISPLAY UNIT   109

POWER SUPPLY
UNIT           110

OPERATING UNIT 107

EP 1 643 126 B1

## FIG. 12

```
        ┌──────────────┐
        │    START     │
        └──────┬───────┘
               │
        ┌──────┴───────┐
        │              │ ── ST1
        │ INITIALIZATION│
        └──────┬───────┘
               │
        ┌──────┴───────┐
        │    CUFF      │ ── ST2
        │ PRESSURIZATION│
        └──────┬───────┘
               │
        ┌──────┴───────┐
        │    CUFF      │ ── ST3
        │ DEPRESSURIZATION│
        └──────┬───────┘
```

ST4 — CUFF PRESSURE MEASUREMENT

ST5 — BLOOD PRESSURE CALCULATION

ST6 — DISCHARGE

ST7 — BLOOD PRESSURE DISPLAY

END

FIG. 13

FIG. 14

$205_1$ — AIR BAG 1

AIR PUMP 1 — $A_1$

DISCHARGE VALVE 1 — $207_1$

$205_2$ — AIR BAG 2

AIR PUMP 2 — $A_2$

DISCHARGE VALVE 2 — $207_2$

$205_n$ — AIR BAG n

AIR PUMP n — $A_n$

DISCHARGE VALVE n — $207_n$

FIG. 15

## FIG. 16A

## FIG. 16B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2551757 B
- JP 3373558 B
- JP 2003193979 A
- JP 2001269375 A
- JP 2003250842 A
- US 20040008853 A1